# EUROPEAN PATENT APPLICATION

(11) **EP 2 027 852 A1**
(43) Date of publication of application: **25.02.2009**
(21) Application number: 07025184.8
(22) Date of filing: 28.12.2007
(51) Int. Cl.: A61K 9/24, A61P 1/02

(54) **Adhesive compositions for the treatment of xerostoma**

(30) Priority: 24.08.2007 US 957899 P
(71) Applicant: Axiomedic Ltd., Gibraltar (GI)
(72) Inventor: Domb, Abraham, J., Efrat 90435 (IL); Brama, Benny, Raanana 43359 (IL)
(74) Representative: HOFFMANN EITLE

(57) **Abstract**

Compositions and methods for the treatment of xerostomia are disclosed herein. Methods for making the compositions are also disclosed herein. The compositions are designed for individuals having the physiologic ability to salivate but who do not salivate in sufficient quantity to satisfy their personal comfort level of oral hydration. The compositions are typically in the form of a film or tablet, preferably in the form of a sticker tablet, most preferably in the form of a double layer sticker tablet, where one layer contains a bioadhesive material and the second layer contains the sialogogic agent and the one or more lipids. The compositions adhere to a buccal surface or mucosal surface in the oral cavity for at least 15 minutes, preferably for at least 30 minutes. The compositions contain a therapeutically effective amount of one or more sialogogic agents and one or more lipids for treating xerostomia and a pharmaceutically acceptable bioadhesive carrier. The compositions optionally contain a non-lipid lubricant, a flavoring agent, and/or a buffering agent. The methods for treatment of xerostomia require placing the composition on the oral mucosa of a patient's mouth, preferably on the palate or the cheek. The composition will adhere to the mucosal surface and dissolve over a period of time. The composition is generally effective at treating or ameliorating the effects of xerostomia for a time period ranging from at least 30 minutes up to 8 hours following administration to the buccal or oral mucosa.

## Description

### FIELD OF THE INVENTION

This invention relates to compositions and methods for the treatment or amelioration of the effects of xerostomia.

### BACKGROUND OF THE INVENTION

Saliva lubricates the mouth and also contains bacteriostatic and digestive principles. Strong tasting and dry foods are the most effective in stimulating this saliva production. Saliva stimulation often follows the consumption of distasteful materials, as a defensive response of the oral mucosa.

Xerostomia (dry mouth) is suffered by an estimated 20% or more of adults, most of whom are women, as a consequence of the inability to secrete saliva. The condition itself is uncomfortable, but may also have serious consequences, resulting in severe dental decay and oral infections. Dry mouth can be caused by a number of maladies, such as Sjogren's syndrome, diabetes, AIDS, bone marrow transplant or dehydration. It may also occur as a response to radiation treatment or as an unwanted side effect of drug treatments. It is thought that over 1,800 drugs have the capacity to cause dry mouth, when taken over a period of time. Dry mouth may also occur as a physiologic response (e.g. stress, nervousness, or "stage fright").

Individuals suffering from Sjögren's syndrome or other pathologic causes of xerostomia lack the ability to salivate. However, individuals suffering from the side effects of drug treatments or from diminished salivary gland activity, may benefit from drug treatments such as pilocarpine, which stimulates salivation as well as other secretions such as sweating. In these cases, the curative treatment may be as objectionable as xerostomia itself due to physiological responses, such as profuse sweating.

Palliative treatments are generally short acting and cosmetic in nature, and include constant ingestion of water, the use of non-cariogenic candies or demulcents, or other agents to directly hydrate the oral cavity or to provide a lubricious mouthfeel. However, individuals' responses to such treatments vary from individual to individual.

There is anecdotal evidence that some traditional herbal or natural products may stimulate salivation. For the most part, these reports concern the chewing of plant materials such as stems, bark, seeds and leaves, etc. Some plant materials used have broadly stimulating properties, such as betel, khat, tobacco and cola nuts. Most plant materials have strong tastes, such as sour tasting lemon and citrus peel, and bitter tasting wormwood, golden seal and yarrow stems, or contain unpleasant-tasting irritants, such as the resins of gum myrrh and the pepper of kava and prickly ash bark.

Prior art oral compositions for the treatment of plaque, which are also capable of stimulating the production of saliva require abrasive materials. For example, U.S. Patent No. 5,804,165 to Arnold discloses an anti-plaque oral composition containing a source of carbon dioxide, silica, and xylitol where the carbon dioxide comes from a bicarbonate. The tablet converts to a solid silica-containing suspension in the saliva of an oral cavity. (*see also* U.S. Patent Nos. 5,817,294; 5,965,110 and 6,086,854, all to Arnold) However, the abrasive materials may harm the dry mucosal tissue, if administered to a patient suffering from xerostomia. Additionally, these compositions only provide s short-term effect.

Bioadhesive sticker tablets for the treatment of oral disdorders, such as ulcers or legions are disclosed in U.S. Publication No. 2007/0104783 to Domb, et al. However, the tablets are not indicated for the treatment of xerostomia.

Treatments of xerostomia have been directed toward the control of dental decay, relief of symptoms and increase of saliva flow. The currently available treatments range from over the counter (OTC) medications to prescription drugs.

Truly effective drugs appear to be few; and a large number of prescription drugs cause or exacerbate xerostomia.

Fluoride gels and rinses are used to increase tooth resistance to caries by promoting remineralization of the teeth. Artificial saliva and saliva substitutes are available in the form of solutions, sprays and lozenges to replace moisture and lubricate the mouth; however, they must be used frequently and consistently as they do not stimulate salivary function.

U.S. Patent No. 5,580,880 to Handa, et al., U.S. Patent No. 5,686,094 to Acharya, U.S. Patent No. 5,962,503 to Ekstrom, et al., and U.S. Patent No. 7,198,779 to Rifa Pinol, et al., disclose compositions containing spirooxathiolane-quinone, polycarbophil, cholinesterase inhibitors, and a combination of saline saliva stimulating substitute agents, saliva production stimulating agent, oral antiseptic and oral mucosal protective agents respectively, for the treatment of xersotomia. However, these compositions do not remain long in the mouth and do not produce a long-lasting effect.

Although there has been some success reported in treating xerostomia, given the large number of patients suffering from xerostomia each year and the larger number of patients undergoing cancer therapy, who often receive multiple cycles of radiation therapy and/or chemotherapy, there is a need for improved treatments for xerostomia. Further, many current treatments provide unpleasant side effects, such as profuse sweating, or require multiple administrations throughout the day to be effective.

It is therefore an object of the invention to provide improved compositions for the treatment of xerostomia.

It is another object of the invention to provide improved methods for the treatment xerostomia.

### SUMMARY OF THE INVENTION

Compositions and methods for the treatment of xerostomia are disclosed herein. Methods for making the compositions are also disclosed herein. The compositions are designed for individuals having the physiologic ability to salivate but who do not salivate in sufficient quantity to satisfy their personal comfort level of oral hydration. The compositions are typically in the form of a film or tablet, preferably in the form of a sticker tablet, most preferably in the form of a double layer sticker tablet, where one layer contains a bioadhesive material and the second layer contains the sialogogic agent and the one or more lipids. The compositions adhere to a buccal surface or mucosal surface in the oral cavity for at least 15 minutes, preferably for at least 30 minutes. The compositions contain a therapeutically effective amount of one or more sialogogic agents and one or more lipids for treating xerostomia and a pharmaceutically acceptable bioadhesive carrier. The compositions optionally contain a non-lipid lubricant, a flavoring agent, and/or a buffering agent. The methods for treatment of xerostomia require placing the composition on the oral mucosa of a patient's mouth, preferably on the palate or the cheek. The composition will adhere to the mucosal surface and dissolve over a period of time. The composition is generally effective at treating or ameliorating the effects of xerostomia for a time period ranging from at least 30 minutes up to 8 hours following administration to the buccal or oral mucosa.

### DETAILED DESCRIPTION OF THE INVENTION

### I. Definitions

"Biocompatible", as generally used herein, means not having toxic or injurious effects on biological function in humans.

"Bioadhesive", as generally used herein, refers to a material which attaches, and preferably strongly attaches, to mucosal tissue upon hydration. The material must be capable of remaining adhered to the tissue in moist or wet *in vivo* environments. The compositions described herein are "self-bioadhesive" in that they attach to the site of interest without the need to reinforce attachment by way of another adhesive material. The strength of adherence can be measured by standard tests for measuring the force, e.g. in dynes per square centimeter, as disclosed in U.S. Patent No. 4,615,697 to Robinson.

"Lipid", as generally used herein, refers to any fat-soluble (hydrophobic) naturally-occurring or man-made molecule.

"Sialogogic agent", as generally used herein, refers to any material that induces and/or stimulates the flow of saliva in a patient's mouth.

"Xerosomia therapeutic agent", as generally used herein, refers to any agent that reduces the symptoms of xerostomia, including by providing comfort to the patient, such as by lubricating the mouth and/or by inducing saliva production. Xerosomia therapeutic agent is a general category that includes sialogogic agents and agents that do not induce saliva production, but improve the patient's comfort. For example, lipids that lubricate the mouth provide comfort to the patient without inducing saliva production. Similarly, hydrogels, such as CARBOPOL® (Lubrizol Advanced Materials, Inc.), retain liquids in the mouth and thereby improve the patient's comfort.

### II. Compositions

The compositions disclosed herein are designed for individuals having the physiologic ability to salivate but who do not salivate in sufficient quantity to satisfy their personal comfort level of oral hydration. The compositions contain a therapeutically effective amount of one or more sialogogic agents and one or more lipids for treating xerostomia and a pharmaceutically acceptable bioadhesive carrier. The compositions optionally contain a buffering compound and/or another xerostomia therapeutic agent, which is neither a sialogogic agent nor a lipid.

The compositions are typically in the form of a film or tablet, preferably in the form of a sticker tablet. The compositions adhere to the mucosal surface in the oral cavity for at least 15 minutes, preferably for at least 30 minutes up to 8 hours following application.

The compositions stimulate the production of saliva in the patient. The compositions further ameliorate dry mouth by delivering a long lasting demulcent property after the initial stimulation of salivation. Typically, the compositions soothe the patient's mouth and maintain a moist oral cavity for at least 30 minutes following administration, preferably for at least 60 minutes following administration, more preferably for at least 3 hours following administration, still more preferably for at least 4 hours following administration, up to 8 hours following administration. up to 8 hours following administration.

Maintenance of a moist oral cavity is important for the preservation of good oral hygiene. A dry mouth is susceptible to infection and other conditions, such as gingivitis, yeast, caries, mucositis, halitosis, and related conditions such as sore throat, and itchy/scratchy throat as in allergic responses, which inadequate mouth pH control and moisture content can exacerbate. Thus, in addition to the direct treatment of xerostomia, these compositions can complement therapeutic approaches to maintaining good oral health.

### a. Dosage Form

The compositions can be in form of a single layer, double layer, or multilayer tablet or film, which are prepared using conventional methods, such as by compression tableting. In one embodiment, the tablet is a single or double layer tablet. In a preferred embodiment, the composition is a double layer bioadhesive film or tablet. The tablet may contain an acceptable plasticizer for the bioadhesive material, and a cohesive agent.

The bioadhesive film or tablet adheres to the buccal or mucosal surface, such as on the palate or cheek, for at least 15 minutes, preferably for at least 30 minutes and adheres for up to 12 hours, preferably for up to 8 hours. Typical time ranges for adherence to the buccal or oral mucosal surface include from 15 minutes to 12 hours and from 15 minutes to 6 hours, preferably from 30 minutes to 8 hours. The film or tablet is designed to deliver the one or more sialogogic agents in combination with the one or more lipids, and optionally any additional xerostomia therapeutic agents included in the composition, for at least 30 minutes, preferably for at least 60 minutes, and for up to 8 hours following administration.

In one embodiment, the composition is a multi-part composition, such as a double layer tablet. Preferably, the sialogogic agent and one or more lipids are contained in a single, relatively homogenous layer in the composition, and the bioadhesive material is contained in a separate, second layer in the composition.

The total mass of the sticker tablet generally ranges from 50 mg to 1000 mg, depending on particular consumer preferences and desired performance attributes such as lozenge residence time (dissolution time) in the mouth. The preferred mass ranges from 100 to300 mg.

The tablets can be of any suitable size for placement in a patient's mouth. In one embodiment, the surface area of the tablet is from about 0.4 cm² to about 3 cm², preferably from about 0.5 cm² to 1.8 cm², more preferably from 0.5 cm² to 1.2 cm². For example, a tablet having a diameter of 15 mm, will have a surface area of approximately 1.8 cm². In the most preferred embodiment, the tablet has a suitable geometry for placement on the desired surface in the mouth. For example, for placement on the palate, the table preferably contains a convex surface designed to be placed adjacent to and adhere to the palate. In a double layer tablet, this side corresponds with the outer surface of the bioadhesive layer of the tablet.

### A. Sialogogic Agents

Sialogogic agents that are useful in the composition include such plant- or herb-based products. The agent may be flavored or flavorless, such as flavorless chemical agents. Sialogogic agents may flavored, but the physiologic response to a sialogogue does not depend upon the presence of a flavor *per se.* Some flavored sialogogic agents are also spices. Additionally, chemicals with no characteristic or distinctive taste may be effective in stimulating salivation. This is the case with chemical agents such as the OPTAMINT®, a food additive based on peppermint extract, or OPTAFLOW®, a food additive contains several flavoring agents and food components.

Flavoring agents can also be used in conjunction with compounds having conventional sialogogic properties. Flavorless chemical sialogogic agents include, but are not limited to, citric acid, citrus oils, and ascorbic acid. Suitable plant- or herb-based sialogogic agents include, but are not limited to cardamom, ginger, licorice, mint extract, and anise.

Sialogogic agents also include pharmaceuticals, such as pilocarpine, cevimeline, anethole trithione, yohimbine, human interferon alpha and amifostine. Pilocarpine is a cholinergic parasympathomimetic agent, which may stimulate salivary flow and produce clinical benefits in some patients as well as cause adverse effects with other drugs. Cevimeline, a cholinergic agonist, is another systemic agent that appears to alleviate xerostomia in certain patient populations. Anethole trithione is a cholagogue that stimulates salivary flow in drug-induced xerostomia. Yolimbine is an alpha-2 adrenergic antagonist that can increase saliva flow. Additional suitable sialogogic agents include sulfur-containing antioxidants, as described in U.S. Publication No. 2007/0128284 to Troha et al.

Sialogogic agents also include biological agents, such as probiotic bacteria, including but not limited to strains of various species of the genera *Bifidobacterium* and *Lactobacillus.*

### Dosage

The sialogogic agent is included in a safe amount to deliver safe (i.e. non-toxic) amounts of the agent as established in the pharmaceutical literature, such as Martindale: The Complete Drug Reference (Pharmaceutical Press). The sialogogic agent should be released from the tablet following administration to the mouth cavity, particularly to the sites where salivary glands are located. The tablet contains a sufficient dose of the sialogogic agent such that the agent is released for the desired time period at an effective amount to treat or alleviate symptoms of xerostomia. The effective amount is typical for each sialogogic agent and the desired release profile is also related to the sialogogic agent can be determined through standard means by one of ordinary skill in the art. The tablet should not contain a dose of the sialogogic agent that is greater than the necessary dose for administration since over dosing may cause patient discomfort or undesirable side effects, such as burning of the mucosal tissue by for example a local overdose of menthol.

Typical doses for biological agents, such as probiotic bacteria, range from about 0.1 microgram to a few milligrams in the tablet or film, depending on the activity of the dry substance and the desired effect.

### B. Lipids

Lipids include fatty-acids and their derivatives (including tri-, di-, and monoglycerides and phospholipids), fatty alcohols and their derivatives, as well as other fat-soluble sterol-containing metabolites such as cholesterol. Triglycerides, include but are not limited to, tricaprin, trilaurin, triacetin, trimyristin, and triolein. Phospholipids, include but are not limited to phosphoglycerides (e.g. phosphatidyl serine, phosphatidyl inositol, phospatidyl ethanolamine, and phosphatidyl choline) and sphingomyelin.

Preferred lipids are generally those that melt at or around body temperature so that they are solid at room temperature, but semiliquid or liquid at body temperature. Examples of preferred lipids include tricaprin, ethyl stearate, short chain waxes, and partially hydrogenated plant oils, such as corn oil. Additional preferred lipids include mixtures or pure mono-, d-i and triglycerides, semisolid phospholipids and hydrophobic short chain polymers, such as polycaprolactone.

The compositions contain an effective amount of the lipid to reduce friction and lubricate the mouth following administration of the composition to a patient suffering from xerostomia. The concentration of the lipid in the composition is at least 5% by weight of the composition, and is no greater than 50% w/w, preferably the composition contains at least 10% (w/w) lipid, preferably the concentration of lipid in the composition ranges from 10% to 30% (by weight).

### Non-Lipid Lubricants

In a preferred embodiment, the composition also includes non-lipid xerostomia therapeutic agents that are not sialogogic agents (referred to as "non-lipid lubricants"). The non-lipid lubricants should be food-grade materials. Suitable non-lipid lubricants include, but are not limited to, hydrogels, such as CARBOPOL® (Lubrizol Advanced Materials, Inc.), water soluble polymers such as polyethylene glycol (m.w 400-1,000,000), glycerol, polypropylene glycol, polyvinyl alcohol, polyvinyl pyrrolidone ("PVP") (e.g. PVP K-30 and/or PVP K-90), sodium benzoate, leucine, magnesium stearate, sodium lauryl sulfate, and sodium lauryl sulfoacetate, in the range of about 3-40% wt/wt.

### C. Bioadhesive Materials

The concentration of the bioadhesive materials in the composition ranges from 5% to 40% by weight of the tablet or film, but can be from 40% to 100% by weight of the adhesive layer in the case of a double layer or multilayer tablet or film, and is preferably from 10-20% by weight of the of the tablet or film. Suitable bioadhesive materials include, but are not limited to, carboxylic acid containing polymers such as copolymers of acrylic or methacrylic acid; esterified polyacrylic acid polymers, such as polyacrylic acid polymers lightly crosslinked with a polyalkenyl polyethers (commercially available from B.F. Goodrich, Cincinnati, Ohio, under the trademarks CARBOPOL® 934, 934P, 974, 940 and 941); maleic acid copolymers; polysaccharides such as karaya gum, tragacanth gum, xanthan gum, jaraya gum, pectin, guar gum, locust bean gum, psyllium seed gum, alginates, hydrocolloid gels prepared from polysaccharides extracted from Fronia elephantum, Sapindus trifoliatus, Kunjac, and the cashew tree; cellulose and cellulose derivatives such as carboxy methyl cellulose, hydroxy propyl cellulose, mixtures thereof, and mixtures of sulfated sucrose and aluminum hydroxide, along with other substances known for use in transdermal preparations capable of forming a solid colloid that can adhere to tissue, used alone or in combination with other suitable carriers.

In a preferred embodiment, the bioadhesive materials contain at least one crosslinked polyacrylic acid, such as CARBOPOL® 934 or 971. In one embodiment, the bioadhesive material is a mixture of crosslinked polyacrylic acid, i.e. CARBOPOL® 940, 934, 974, 971, carboxymethyl cellulose (CMC) and hydroxypropylmethyl cellulose (HPMC).

### D. Buffering Compounds

The composition typically includes one or more buffering compounds in an effective amount to maintain a neutral pH in the oral cavity for at least 30 minutes following administration. As the composition dissolves in the oral cavity, it generates a buffered solution in the oral cavity which, by maintaining a relatively neutral pH, can help to diminish the formation of caries and renders the oral cavity less prone to infection.

Preferred buffering compounds include disodium hydrogen phosphate, calcium chloride, citric acid, sodium citrate or potassium citrate, sodium acetate, ethanol amine, or a combination thereof. Other suitable buffering compounds include acids, such as fumaric acid, tartaric acid, malic acid, adipic acid, and other edible acids or their pharmaceutically acceptable salts can be used. Sodium carbonate and sodium bicarbonate are the preferred carbonate salts. However carbonates and bicarbonates of potassium, sodium, ammonium, magnesium, calcium can also be used. The composition may contain from about 1% to about 10% by weight of the buffereing compound.

Preferably the composition includes one or more excipients, such as glycerin, polymers such as (but not limited to) natural gums including Xanthan (0.5 to 15% by weight), and 0.2 to 10% by weight of cellulose based materials such as methylcellulose, carboxymethylcellulose, hydroxypropylmethyl cellulose, hydroxyethylcelulose), acrylic acids at 0.2 to 10% by weight, polyethylene glycols at 0.2 to 2 wt %, dextran (0.05 to 0.5 wt. %), gelatin (0.2 to 5 wt. %), polyvinyl alcohol (0.1 to 5 wt. %), polysorbate 80 (0.2 to 2 wt. %), or povidone (0.1 to 4 wt. %), which increases the time period during which the buffered solution remains in the oral cavity, as compared to the same composition in the absence of such excipients.

### D. Carriers, Additives and other Excipients

### Carriers

Compositions are prepared using a pharmaceutically acceptable carrier composed of materials that are considered safe and effective and may be administered to an individual without causing undesirable biological side effects or unwanted interactions. The term "carrier" includes, but is not limited, to diluents, binders, stabilizers, flavoring agents, pigments, humectants, disintegrators, and fillers.

### Humectants

Suitable humectants include, but are not limited to, edible polyhydric alcohols, such as glycerin, sorbitol, xylitol, butylene glycol, polyethylene glycol, and propylene glycol. In one embodiment, the humectant is sorbitol and/or glycerin. The humectant may also act as a plasticizer to provide a flexible sticker, which is comfortable to the user when placed in his/her mouth. The concentration of the humectant is from about 1% to about 20% by weight of the composition, preferably from about 1% to about % by weight of the composition.

### Flavoring agents

Preferably the composition contains one or more flavoring agents, such as natural or artificial sweeteners. This is particularly preferred if the sialogogic agent is flavorless or has an unpleasant-taste.

Suitable flavoring agents include, but are limited to, oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, thymol, linalool, cinnamaldehyde glycerol acetal known as CGA, and combinations thereof. The concentration of the flavoring agent is from about 0.001% to about 1% by weight of the composition. Some of the compounds listed as excipients and carriers can also be active agents depending on the concentration of the compound in the composition. For example, menthol at a concentration of 0.01% by weight is typically a flavoring agent but at a concentration of 2.0% by weight can be an active agent.

Natural or artificial sweeteners include, but are not limited to, sucrose, glucose, saccharin, dextrose, levulose, lactose, mannitol, sorbitol, fructose, maltose, xylitol, saccharin salts, thaumatin, aspartame, D-tryptophan, dihydrochalcones, acesulfame and cyclamate salts, especially sodium cyclamate and sodium saccharin, and combinations thereof. Preferably the sweetener is a non-cariogenic sugar alcohol. The concentration of the sweetener is from about 0.1 % to about 2% by weigh of the composition, preferably from about 0.1 % to about 1% by weight of the composition.

Flavoring agents (including sweeteners) are present in the tablet at 0.1% to 5% by weight, depending on the specific flavor and desired attributes. If plant-based or herb-based silialogic agents are incorporated into the compositions, these may be included at 0.25% to 20% of the composition by weight. Artificial sweeteners are used according to taste, and generally the composition contains at least 0.1% (wt/wt) of an artificial sweetener.

### Binders

Tabletting materials such as binders, fillers, and flow aids typically accounts for about 90% of the mass, and comprises mostly sugar alcohols, but includes flow aids, lubricants, flavors and excipients. Suitable binders include sorbitol, lactose, urea, sucrose stearate, starch, maltodextrin, corn syrup solids, sodium citrate, sodium sulfate, sodium chloride, sucrose, and dextrates.

### Additional Additives

Desensitizing agents may also be added, such as strontium nitrate and potassium nitrate, to reduce heat or cold sensitivity.

Antimicrobial agents such as cetylpyridinium chloride and domiphen bromide may be added to reduce bacterial levels in the oral cavity.

Breath freshening ingredients such as chlorophyll may be added and pharmaceutical agents such as antibiotics may also be included in the compositions.

Non-limiting examples of some other components that may be included in a delivery composition include one or more of the following: penetration enhancers, stabilizers for the xerostomia therapeutic, preservatives such as antioxidants butylated hydroxytoluene, antifungals, and antibacterials.

### E. Preferred Tablet

In the preferred embodiment, the composition is a double layer bioadhesive tablet, where one layer contains a bioadhesive material and the second layer contains the sialogogic agent and one or more lipids. Preferably the tablet is shaped to be placed in the palate or cheek, such as by having a convex surface on the side containing the bioadhesive material to facilitate placement and adherence to the desired mucosal surface.

In one preferred embodiment, the sialogogic agent is a hydrophilic compound, preferably an acid, such as citric acid or ascorbic acid, and the lipid is a fatty ester.

In a preferred embodiment, the bioadhesive sticket tablet or film contains 10-20 wt. % CARBOPOL® 934 or CARBOPOL® 971 (as a non-lipid, non-sialogogic xerostomia therapeutic agent), 1-4 wt. % calcium chloride, 10 - 40 wt. % tricaprin or trilaurin (as the lipid component), 10-40 wt. % non-cariogenic sugar alcohol (as a flavoring agent), 0-8 wt. % citric acid (as the sialogogic agent), 1-3 wt. % sodium bicarbonate, 5-40 wt. % binder, and 1-15 wt. % polymer or gum (as a lubricant).

### III. Methods of Administering the Compositions

The sticker tablet is applied to the oral mucosa to treat xerostomia. Preferably the tablet is placed on the palate of the mouth cavity; however it may be placed in any suitable mucosal tissue inside the mouth, such as on the cheek The bioadhesive sticker tablet adheres to the oral mucosal tissue and provides relief for at least 30 minutes. The bioadhesive sticker tablet adheres to the oral mucosa for at least 15 minutes, preferably for at least 30 minutes, preferably from about 1 hour to about 8 hours, more preferably from about 1 hour to about 4 hours.

The compositions dissolve after placement in the mouth, typically over a time period ranging from 30 minutes to 8 hours.

The sticker tablet is preferably administered from about once a day to four times per day, more preferably from about once per day to about twice per day. In some treatments, the patient will rest for a suitable period of time between applications of the tablet or film to allow natural activity of the mouth organs. Additionally or alternatively, in the preferred embodiment, the tablet should be attached in different locations in the patient's mouth for consecutive treatments to minimize local mucosal irritation and provide relief for the immediately prior adhesion site. For example, one tablet may be placed onto a first location, such as the palate; for the second treatment, the tablet may be placed in a different location, such as on the buccal mucosa, and for a subsequent treatment, the tablet may be placed at the first location (e.g. the palate) or a location different from both the first and second placement locations.

Tablets of different compositions and/or containing different silialogogic agents may be placed in the mouth to allow different stimulations of the salivary glands or effects on mucosal tissues.

In severe cases of xerostomia, or when small tablets are used, two or more tablets can be places in different locations simultaneously. However, typically treatment will require placement of one tablet at a time in the mouth.

The tablets are administered to treat and/or reduce the severity of xerostomia, such as by ameliorating symptoms associated with xerostomia, compared to no treatment. It is believed that the tablets are effective at treating or ameliorating the severity of or symptoms associated with xerostomia by protecting superficial salivary function, stimulating salivary flow and/or decreasing the viscosity of saliva.

### IV. Method of Making the Compositions

### Compression Molding

Compression molding can be used to prepare single layer, dual layer, or multilayer sticker tablets. The simplest method for preparing the sticker tablets is by compression molding using a single or multi-punch press machine. The powder is loaded in the punch having a diameter ranging from about 4 to about 15 mm and a thickness of about 0.5 mm to about 2.5 mm. The thickness is defined by the amount of powder added, usually between about 50 mg and 250 mg. The powder is compressed to form a single layer sticker tablet

Double layer sticker tablets are prepared using the double compression technique. The inert powder is first added to the punch to cover the surface. The formulation powder is added on top and compression is applied to produce a sticker tablet where one side is bioadhesive and the other is not. The non-bioadhesive side also tends to be less water-permeable than the bioadhesive side. Alternatively, one powder is added to the punch and compressed to form a thin tablet. The second powder is then added and compressed to form a uniform bilayer tablet.

### Spray Coating

Double layer sticker tablets can also be prepared by spray coating. In the spray coating method, the coating is applied by spraying an alcoholic solution or fine dispersion of a hydrophobic coating material onto one side of the sticker tablet. The spray coating can be applied using an automated machine where the tablets are placed onto a running sheet which is exposed to spray nozzles to spray coat the tablets. Typical hydrophobic powders suitable for this coating include: fatty acids and salts such as Mg- or Ca-stearate, triglycerides and fatty acid esters, ethyl cellulose, methyl methacrylate-methacrylic acid copolymers (EUDRAGIT®), and other pharmaceutically acceptable hydrophobic components.

### Solvent casting

Another way of preparing thin single layer sticker tablets is by casting a concentrated suspension in ethanol of all tablet ingredients onto a flat surface where, after solvent evaporation, a thin sheet is obtained. The sheet is then cut into films of the desired size and shape using a cutting mold.

Double layer films can be prepared by applying the coating as a spray on top of the sheet loaded with the active agents. Other industrial methods can be used, such as forming the sheet on an edible hydrophobic sheet such as rice paper and cutting the sheets into the desired size.

### Controlled Release Compositions

In some embodiments, the tablet or film is designed for controlled release of the sialogogic agent.

In one embodiment, a sialogogic agent is absorbed in or onto a polymeric component or is encapsulated into microcapsules that control the release of the agent when embedded in the tablet. For example, a solution (e.g. ethanolic solution) of a sialogogic herbal extract may be absorbed in a polymer matrix, such as crosslinked polyacrylic acid (e.g. CARBOPOL®), hydroxyl propyl cellulose (HPC), ethyl cellulose or EUDRAGIT® powders, and added to the tablet mixture, preferably on the outer layer.

Encapsulation of sialogogic agents in matrix type or capsule type particles can be done via standard methods used in the pharmaceutical industry. Encapsulating materials include, but are not limited to, ethyl cellulose, copolymers of methacrylic acid and methyl methacrylate, gelatin, alginates, gum polysaccharides, polycyanoacrylate, etc.

Encapsulation processes are selected or designed taking into consideration the heat sensitivity and the low melting or boiling point and/or the sublimation temperature of the sialogogic agents of interest.

### Inclusion of Biological agents in Tablet or Film

When biological agents, such as probiotic agents and enzymes, are included in the tablet or film, they are typically incorporated as a dry powder. Prior to the addition of the biological agents to the other materials for the formation of the tablet or film, the biological agents may be stabilized with a mixture of amino acids, salts, and acidic and basic small molecules, may be encapsulated in a polymeric carrier or may be absorbed in a pharmaceutically acceptable excipient or additive, such as polysaccharides or acrylate-based polymers.

### EXAMPLE

### Example 1: Research and Clinical trials testing friability, dissolution time and efficacy for bioadhesive tablets for treatment of xerostomia

### Compositions and Methods of Manufacture

Eight (8) bioadhesive double-layer sticker tablets containing different amounts of lipid (i.e. Tricaprin), sialogogic agent, etc., as detailed in Table 1, were prepared.

The first layer in the tablet was an adhesive layer that contained 30 mg of Carbopol 940 and 10 mg of hydroxy propyl cellulose (HPC). The second layer was formed from a mixture of polyvinylpyrrolidone (PVP), xylitol, tricaprin, lemon flavor and CARBOPOL® 940. The tablet was prepared in two steps, and the adhesive layer was placed on top of the second layer containing the sialogogic agent. The total weight for each tablet was about 240 mg.

The second layer also contained a pink dye commonly used in food to distinguish this layer from the first, bioadhesive layer. Also, the tablet was convex on the side containing the adhesive layer and flat on the side containing the layer containing the sialogogic agent. This design enabled the table to fit snugly on the palate in the mouth.

**Table 1: Composition of Layer containing the Sialogogic Agent in Bioadhesive Double-layer Sticker Tablets**

| | **Tablet Number** | | | | | | | |
|---|---|---|---|---|---|---|---|---|
| **Composition** | **1** | **2** | **3** | **4** | **5** | **6** | **7** | **8** |
| Calcium chloride | | | | | | | | |
| (%w/w) | 5 | 5 | 5 | 5 | 5 | 5 | 5 | 5 |
| Xylitol (%w/w) | 44.5 | 44.5 | 44.5 | 33.5 | 44.5 | 38.5 | 32.5 | 26.5 |
| PVP K-90 | | | | | | | | |
| (%w/w) | - | 30 | 15 | 51 | 30 | 26 | 22 | 18 |
| PVP K-30 | | | | | | | | |
| (%w/w) | 30 | - | 15 | - | - | - | - | - |
| Tricaprin (%w/w) | 20 | 20 | 20 | 10 | 20 | 30 | 40 | 50 |
| Trilaurin (%w/w) | - | - | - | - | - | - | - | - |
| Lemon flavor | | | | | | | | |
| (%w/w) | 0.5 | 0.5 | 0.5 | 0.5 | 0.5 | 0.5 | 0.5 | 0.5 |
| Total (%w/w) | 100 | 100 | 100 | 100 | 100 | 100 | 100 | 100 |

The components in the non-adhesive layer included:
- polyvinylpyrrolidone (PVP) (K-value of 30 or 90) - a binding agent with hydrophilic lubricating affect (i.e. a non-lipid lubricating agent),
- xylitol - a sweetener and humectant,
- tricaprin - lipid that melts at body temperature,
- lemon flavor - a sialogogic agent, and
- calcium chloride - a buffering agent.

### Friability and Dissolution Test

For the fiability and dissolution tests, three (3) tablets were tested for each tablet number. Friability tests were performed by using a universal friability test and according to the USP (Ref: 23, page 1981, as described in Remington Pharmaceutical Technology Handbook). Time of dissolution was determined by placing one tablet in a vial followed by the addition of 15ml buffer phosphate (pH 6.5) at 37°C. Buffer was replaced every 10 minutes with fresh buffer. Results for the friability and dissolution tests are provided in Table 2.

**Table 2: Results of Friability and Dissolution Tests**

| **Tablet No.** | **Short Name for Tablet** | **Friability** | **Time of dissolution (min)** |
|---|---|---|---|
| 1 | 30% PVP - K30 | 0.6 ± 0.1 | 10.5 ± 1 |
| 2 | 30% PVP - K90 | 0.26 ± 0.05 | 27.5 ± 2.5 |
| 3 | Mixture PVP K30:K90 (1:1) | 0.35 ± 0.04 | 52.5 ± 2.5 |
| 4 | 10% lipid | 0.26 ± 0.11 | 52.5 ± 2 |
| 5 | 20% lipid | 0.26 ± 0.05 | 49 ± 1 |
| 6 | 30% lipid | 0.16 ± 0.05 | 43 ± 1 |
| 7 | 40% lipid | 0.23 ± 0.03 | 24.5 ± 1 |
| 8 | 50% lipid | 0.62 ± 0.12 | 10.5 ± 1 |

As shown in Table 2, Tablet 1, which contained 30% (wt/wt) PVP K-30 as a non-lipid lubricating material, was more friable and dissolved much faster than Tablets 2 and 3, which contained 30% (wt/wt) PVP K-90 and 30% (wt/wt) of a 1:1 mixture of PVP K-30 and PVP K-90, respectively.

Further, Tablets 4-6, which contained 10%, 20% and 30% (wt/wt) lipid, respectively had approximately the same friability and similar dissolution times. Although Table 7, which contained 40% (wt/wt) lipid had a similar friability to Tablets 4-6, it dissolved much more quickly (within about 24.5 minutes). Finally, Tablet 8, which contained the highest amount of lipid was also the most friable and dissolved the fastest of Tablets 4-8 (within about 10.5 minutes). Tablets 5 and 6 were selected for further research and development.

### Clinical Study

### Patients

Double layer sticker tablet having the composition of Tablet 5 (*see* Table 1) were tested in the following clinical study. The clinical study was conducted in the School of Dentistry, at The Hebrew University, Jerusalem, Israel. The protocol was approved by the institutional review board of Hadassah Hospital. 22 patients were tested. All patients gave written informed consent before entry and before study-related procedures were performed.

Eligible xerostomic patients were at least 18 years of age, and suffered from dry mouth syndrome due to Sjogren's syndrome or due to previous treatment with head and neck radioiodine, or complained of dry mouth. Patients suffering from glaucoma, cardiac arrhythmias, pulmonary, bladder problems, or an autoimmune disease were excluded from the study. The mean age of the patients was 53 years and the age range was from 18 years to 72 years.

The study was conducted between 8:00 and 12:00 am, after 2 hours of free of food intake, mouthwash use or tooth brushing. Whole salivary flow was collected for 10 minutes before and at 1, 2, 3, and 6 hours after the application of one adhesive tablet. The adhesive tablet was placed by a dentist in the palate of the patient's mouth. Only one palate was placed in the mouth in this study. Systolic and diastolic blood pressure, heart rate, and body temperature were measured at each time point. The study was continued for 5 hours, during which time the patient's complaints and subjective feelings were recorded.

A control group of 10 patients was instructed to use a commercial spray (BIOTENE®, Laclede Professional Products, Inc. Rancho Dominguez, CA 90220 USA) about 3 to 5 times during the 5-hour long study. BIOTENE® mouthwash contains four antibacterial enzymes which boost the defense system normally found in the saliva.

Saliva sampling and pH measurement for various sites in the oral cavity were performed by one trained clinician, who was blinded to treatment sequence, before and after each treatment. On each occasion, sampling was performed in the morning to minimize bias due to diurnal variations in salivary flow. Unstimulated whole saliva (UWS) was collected by spitting any saliva into a sterile, pre-weighed container over a period of 5 minutes. Stimulated whole saliva (SWS) was collected by having the subject chew on a standard piece of sterilized silicone rubber tubing for 5 minutes and spitting all saliva into a sterile, pre-weighed container.

The weight of the UWS and SWS was recorded and the salivary flow rate determined and expressed as ml/min.

Patient satisfaction with aspects of oral comfort and function during the two treatments was explored by asking questions on mouth condition, speaking, chewing hard and soft foods, swallowing and comfort of the mouth. In addition, specific questions about ease of use of the Tablet treatment and the Control treatment were asked.

Responses were made using 100 mm Visual Analog Scale ("VAS") with anchor words: extremely difficult/no difficulty and totally unsatisfied/totally satisfied, as appropriate.

### Statistical Analysis

Statistical comparisons of the findings were made by one-way analysis of variance. Comparison of means was performed by the least significant difference test. Data analysis was performed using a statistical software package (Instat; GraphPad Software, San Diego, CA). The significance level was set at p<0.05.

### Results

The single-layer bioadhesive tablets improved objective and subjective scores compared to treatment with the control. The administration of the single-layer bioadhesive tablets reduced the severity of xerostomia symptoms compared to the control and was the treatment of choice compared to the control.

A significant increase (p=0.03) in salivary flow rate was observed immediately (2-5 minutes) after adhering one tablet. Also after 1, 2, and 5 hours base line level of salivary flow was higher (p=0.03) following application of the tablet compared to following treatment with the control.

Control performance was 50% lower than results achieved following administration with the tablet in objective results, including saliva flow measurements. Whole saliva pH increased significantly among the patients given tablets (p=0.00) comparing to the control (p=0.04).

Patients' satisfactory and overall compliance was high for both treatments. However, the overall patient satisfaction score was higher for the tablet treatment compared to the control, and the majority of participants chose tablets as the preferred treatment.

Unless defined otherwise, all technical and scientific terms used herein have the same meanings as commonly understood by one of skill in the art to which the disclosed invention belongs. Publications cited herein and the materials for which they are cited are specifically incorporated by reference.

Those skilled in the art will recognize, or be able to ascertain using no more than routine experimentation, many equivalents to the specific embodiments of the invention described herein. Such equivalents are intended to be encompassed by the following claims.

## Claims

1. A bioadhesive sticker tablet for treating or ameliorating the effects of xerostomia in a patient comprising a sialogogic agent and one or more lipids in a therapeutically effective amount to treat or ameliorate the effects of xerostomia, and a pharmaceutically acceptable bioadhesive carrier, wherein the tablet comprises at least 5% (wt/wt) of the one or more lipids, and
wherein the tablet, upon application to a mucosal surface, adheres to the mucosal tissue for at least 15 minutes.

2. The tablet of claim 1, wherein the tablet is in the form of a single layer tablet.

3. The tablet of claim 1, wherein the tablet is in the form of a double layer tablet.

4. The tablet of claim 3, wherein the double layer layer tablet comprises a first layer comprising sialogogic agent and one or more lipids and a second layer comprising one or more bioadhesive materials.

5. The tablet of claim 1, wherein the tablet dissolves following application to the mucosal surface over a period of time ranging from 15 minutes to 8 hours.

6. The tablet of claim 1, further comprising a flavoring agent, a binder, or a buffering agent, or a combination thereof.

7. The tablet of claim 1, further comprising a non-lipid lubricant.

8. The tablet of claim 1, wherein the one or more lipids are selected from the group consisting of fatty-acids and their derivatives, fatty alcohols and their derivatives, and other fat-soluble sterol-containing metabolites.

9. The tablet of claim 8, wherein the one or more lipids are selected from the group consisting of triglycerides and phospholipids.

10. The tablet of claim 1 wherein the sialogogic agent is a plant-based sialogogic agent selected from the group consisting of cardamom, ginger, licorice, mint extract, and anise.

11. The tablet of claim 1, wherein the sialogogic agent is selected from the group consisting of citric acid, citrus oils, ascorbic acid, and probiotic bacteria.

12. A method for treating or ameliorating the effects of xerostomia in a patient, comprising:
placing on the buccal or oral mucosa of the mouth of the patient an adhesive tablet comprising a sialogogic agent and one or more lipids in a therapeutically effective amount to treat or ameliorate the effects of xerostomia, and a pharmaceutically acceptable bioadhesive carrier, wherein the tablet comprises at least 5% (wt/wt) of the one or more lipids.

13. The method of claim 12, wherein the tablet is placed on the palate or the cheek.

14. The method of claim 12, wherein the tablet adheres to the buccal or oral mucosa for at least 15 minutes.

15. The method of claim 12, wherein the tablet further comprises a non-lipid lubricant.

16. The method of claim 12, wherein the one or more lipids are selected from the group consisting of fatty-acids and their derivatives, fatty alcohols and their derivatives, and other fat-soluble sterol-containing metabolites.

17. The method of claim 16, wherein the one or more lipids are selected from the group consisting of triglycerides and phospholipids.

18. The method of claim 12, wherein the sialogogic agent is a plant-based sialogogic agent selected from the group consisting of cardamom, ginger, licorice, mint extract, and anise.

19. The method of claim 12, wherein the sialogogic agent is selected from the group consisting of citric acid, citrus oils, ascorbic acid, and probiotic bacteria.
